# EUROPÄISCHE PATENTSCHRIFT

(11) **EP 1 459 720 B1**
(45) Veröffentlichungstag und Bekanntmachung des Hinweises auf die Patenterteilung: **27.04.2005**
(21) Anmeldenummer: 03022429.9
(22) Anmeldetag: 07.10.2003
(51) Int. Cl.: A61F 13/20

(54) **Vaginal-Tampon**
Vaginal tampon
Tampon hygiénique

(30) Priorität: 18.02.2003 DE 10306678
(43) Veröffentlichungstag der Anmeldung: 22.09.2004
(73) Patentinhaber: Hysalma GmbH, 45481 Mülheim an der Ruhr (DE)
(72) Erfinder: Schmidt, Lothar, 09569 Oederan (DE)
(74) Vertreter: COHAUSZ DAWIDOWICZ HANNIG & PARTNER

(56) Entgegenhaltungen:
- EP-A- 1 108 408
- EP-A- 1 125 570
- US-A- 5 374 258
- US-A1- 2002 151 859
- US-A1- 2002 157 222

## Beschreibung

Die Erfindung betrifft einen Vaginal-Tampon mit längs des Tampons in seiner Außenseite angeordneten Rillen (Nuten), die längs angeordnete Rippen voneinander trennen.

Es sind Vaginal-Tampons bekannt, deren Rillen gerade oder spiralförmig um den Tampon herum verlaufen. Bei diesen bekannten Tampons hat es sich gezeigt, dass die Flüssigkeitsaufnahme ungenügend und nicht schnell genug erfolgt.

Aufgabe der Erfindung ist, durch gezielte Veränderung der Ausbildung der Längsrippen die Oberfläche des Tampons gegenüber den bisher bekannten Ausführungen gezielt zu vergrößern.

Dies wird durch die Ausbildung der Rillen und Rippen in einer Wellenform erreicht, die sich parallel zur Längsachse orientiert.

Die Form der Wellen und die Häufigkeit, mit der die Mäanderkurve die imaginäre Längsachse schneidet, ist in weiterem Rahmen frei wählbar.

Die Fasermasse, die im wesentlichen die Kapazität zur Aufnahme der Körperflüssigkeit bestimmt, wird wesentlich besser ausgenutzt.

Die Saugeigenschaften des Tampons kann den Bedingungen in der Körperhöhle besser angepasst werden.

Der Tragekomfort des Tampons erhöht sich und das Abdichtverhalten verbessert sich, da durch die Mäanderform der Rippen und Rillen sich der Tampon bei seiner Ausdehnung der Körperhöhle besser anpasst.

Die Mäanderform der Rillen und Rippen erlaubt eine gezielte Vergrößerung der wirksamen Produktoberfläche. Gerade Rillen und Rippen sind aus geometrischen Gründen nicht geeignet, die Oberfläche in gegebenen Tamponabmessungen zu vergrößern. Spiralförmig angeordnete Rillen und Rippen sind aus produktionstechnischen Gründen in der Vergrößerung der wirksamen Oberfläche begrenzt.

Die Herstellung des erfindungsgemäßen Produkts ist mit Werkzeugen möglich, die ohne Hinterschneidung radial in den Rohwickel des Tampons eintreten. Bei der Ausformung des erfindungsgemäßen Produkts werden die erzeugten Rillen und Rippen nicht wieder teilweise zerstört.

Es können erfindungsgemäß verschiedene Rillentiefen realisiert werden, um so gezielt die Körperflüssigkeit in den Tampon einzuleiten, bei Gewährleistung der notwendigen Produktstabilität (Knickstabilität).

Erfindungsgemäß kann der Verdichtungsgrad des gepressten Tampons in Abhängigkeit zu den verwendeten Fasern gezielt angepasst werden. Die mechanischen Eigenschaften des Tampons, die Knickstabilität und das Expansionsverhalten, können erfindungsgemäß durch die Ausbildung der Wellen beeinflusst werden.

Die erfindungsgemäße Ausführung der Rillen und Rippen in Wellenform erlauben es, die Flüssigkeitsaufnahme des Tampons gezielt anzupassen. Dies bedeutet zum Beispiel am Tampon-Kopf eine wesentlich engere Wellenfolge als am Tampon-Ende. Dies unterstützt die Aufnahme der Körperflüssigkeit in der Körperhöhle und gleichzeitig die Verbesserung der Leckageeigenschaften.

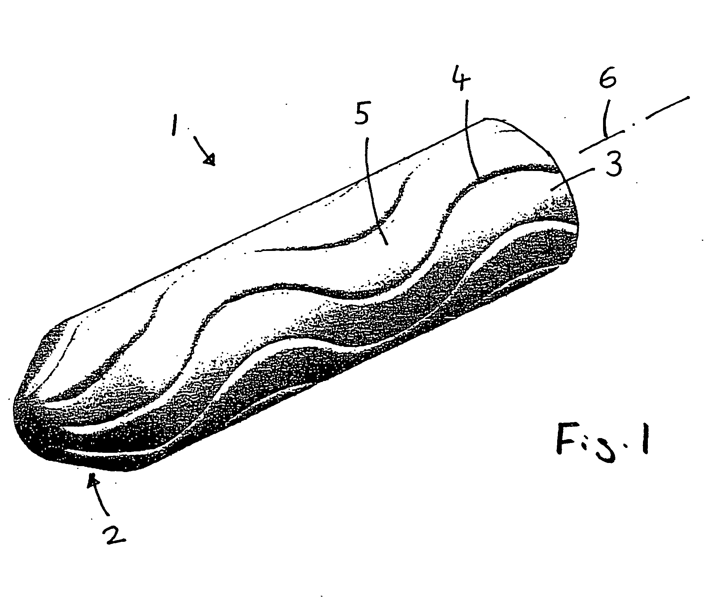

Drei vorteilhafte Ausgestaltungen der Erfindung sind in den Zeichnungen dargestellt und werden im folgenden näher beschrieben. Es zeigen
- Fig. 1: ein erstes Ausführungsbeispiel mit wellenförmigen Rillen,

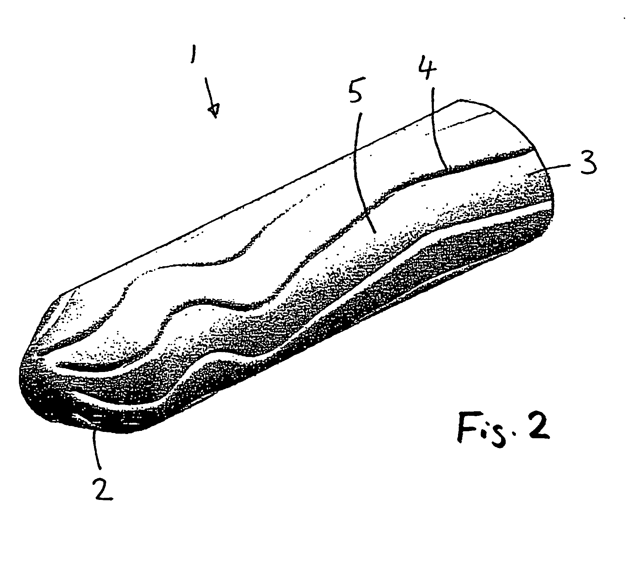

- Fig. 2: ein zweites Ausführungsbeispiel mit wellenförmigen Rillen in stärkeren Wellensteigungen zu Beginn des Tampons,

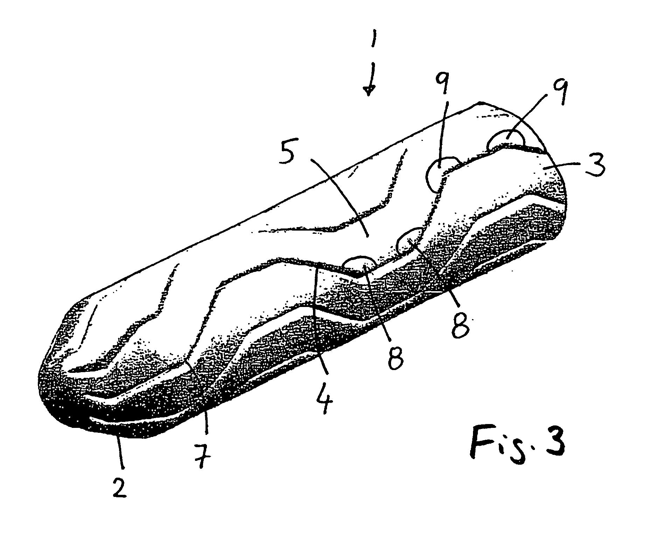

- Fig. 3: ein drittes Ausführungsbeispiel mit Knickstellen aufweisenden Wellen.

Die Erfindung betrifft einen Vaginal-Tampon 1 für die Frauenhygiene, mit einem runden und/oder einem konischen Einführende bzw. Einführbereich 2 und einem Rückholende 3, an dem ein Rückholfaden heraustritt.

Der Tampon 1 wird aus einem Rohwickel gepresst. Der Rohwickel besteht aus flüssigkeitsabsorbierenden Fasern, einer Umhüllung aus einem flüssigkeitsdurchlässigen Material und dem eingelegten Rückholfaden.

Durch die besondere Ausführung der herstellenden Presswerkzeuge werden Längsrillen 4 bei dem Pressvorgang in den Tampon eingebracht. Diese Längsrillen bzw. Längsnuten vergrößern die im wesentlichen zylindrische Oberfläche des Tampons und bilden zusätzlich Eindringkanäle für die zu absorbierende Körperflüssigkeit.

Zwischen den eingepressten Längsrillen 4 bilden sich Längsrippen 5. Diese Längsrippen geben dem Tampon die notwendige Stabilität. Die Längsrillen 4 und Längsrippen 5 sind radial zum Mittelpunkt des Tampons gerichtet und an der Tampon-Längsachse 6 orientiert.

Beim erfindungsgemäßen Tampon sind bis auf den vorderen Einführbereich 2 die Abstände der Rillen 4 voneinander gleich groß und die Wellenform weist Krümmungsbereiche mit unterschiedlich großen Radien auf.

Ferner weist die Wellenform abwechselnd Bereiche stärkerer und geringerer Krümmung auf. Hierbei kann die Wellenform Knickstellen aufweisen.

Die Wellenform des Tampons ist abwechselnd aus Paaren stumpfer 8 und überstumpfer Winkeln 9 zusammengesetzt, ist zum Tampon-Ende hin flacher und weist somit weniger starke Krümmungen auf.

## Patentansprüche

1. Vaginal-Tampon (1) mit längs des Tampons in seiner Außenseite angeordneten Rillen (Nuten) (4), die längs angeordnete Rippen (5) voneinander trennen, **dadurch gekennzeichnet, dass** die Rillen (4) und Rippen (5) bei ihrer Längserstreckung wellenförmig sind.

2. Tampon nach Anspruch 1, **dadurch gekennzeichnet, dass** bis auf den vorderen Einführbereich (2) des Tampons die Abstände der Rillen (4) voneinander gleich groß sind.

3. Tampon nach Anspruch 1 oder 2, **dadurch gekennzeichnet, dass** die Wellenform Krümmungsbereiche mit unterschiedlich großen Radien aufweist.

4. Tampon nach einem der vorherigen Ansprüche, **dadurch gekennzeichnet, dass** die Wellenform abwechselnd Bereiche stärkerer und geringerer Krümmung aufweist.

5. Tampon nach einem der vorherigen Ansprüche, **dadurch gekennzeichnet, dass** die Wellenform Knickstellen (7) aufweist.

6. Tampon nach Anspruch 5, **dadurch gekennzeichnet, dass** die Wellenform abwechselnd aus Paaren stumpfer (8) und überstumpfer Winkeln (9) zusammengesetzt ist.

7. Tampon nach einem der vorherigen Ansprüche, **dadurch gekennzeichnet, dass** die Wellenform zum Tampon-Ende hin flacher ist und somit weniger starke Krümmungen aufweist.

## Claims

1. Vaginal tampon (1) having furrows (grooves) (4) arranged longitudinally of the tampon in the exterior thereof which separate longitudinally arranged ribs (5) from one another, **characterised in that**, as they extend longitudinally, the furrows (4) and ribs (5) are of a wavelike shape.

2. Tampon according to claim 1, **characterised in that**, except at the front insertion region (2) of the tampon, the spacings of the ribs (4) from one another are uniform in size.

3. Tampon according to claim 1 or 2, **characterised in that** the wavelike shape has curved regions of different radii.

4. Tampon according to one of the foregoing claims, **characterised in that** the wavelike shape has alternating regions of greater and lesser curvature.

5. Tampon according to one of the foregoing claims, **characterised in that** the wavelike shape has angled points (7).

6. Tampon according to claim 5, **characterised in that** the wavelike shape is made up of alternating pairs of obtuse (8) and reflex (9) angles.

7. Tampon according to one of the foregoing claims, **characterised in that** the wavelike shape is flatter towards the end of the tampon and thus has less pronounced curves.

## Revendications

1. Tampon hygiénique (1) avec des gorges (rainures) (4) disposées le long du côté extérieur du tampon, qui séparent entre elles des nervures (5) longitudinales, **caractérisé en ce que** les gorges (4) et les nervures (5) sont ondulées dans leur extension longitudinale.

2. Tampon suivant la revendication 1, **caractérisé en ce que** les écartements des gorges (4) sont égaux entre eux exception faite de la zone d'introduction avant (2) du tampon.

3. Tampon suivant l'une des revendications 1 et 2, **caractérisé en ce que** la forme ondulée présente des zones de courbure de rayons différemment élevés.

4. Tampon suivant l'une des revendications précédentes, **caractérisé en ce que** la forme ondulée présente en alternance des zones de courbure plus forte et plus faible.

5. Tampon suivant l'une des revendications précédentes, **caractérisé en ce que** la forme ondulée présente des points d'inflexion (7).

6. Tampon suivant la revendication 5, **caractérisé en ce que** la forme ondulée est composée en alternance de paires d'angles obtus (8) et d'angles concaves (9).

7. Tampon suivant l'une des revendications précédentes, **caractérisé en ce que** la forme ondulée est plus plate en direction de l'extrémité du tampon et présente ainsi des courbures moins fortes.
